# EUROPEAN PATENT SPECIFICATION

(11) **EP 2 491 951 B1**
(45) Date of publication and mention of the grant of the patent: **20.09.2017**
(21) Application number: 10824891.5
(22) Date of filing: 18.10.2010
(51) Int. Cl.: A61K 47/32, A61K 9/10, A61K 31/337, A61K 31/4375, A61K 47/34, A61P 1/04, A61P 1/16, A61P 1/18, A61P 13/02, A61P 13/12, A61P 15/00, A61P 35/00, A61K 9/107, A61K 9/00

(54) **BLOCK COPOLYMER FOR INTRAPERITONEAL ADMINISTRATION CONTAINING ANTI-CANCER AGENT, MICELLE PREPARATION, AND CANCER THERAPEUTIC AGENT COMPRISING THE MICELLE PREPARATION AS ACTIVE INGREDIENT**
BLOCKCOPOLYMER FÜR INTRAPERITONEALE VERABREICHUNG MIT EINEM ANTIKREBSMITTEL, MIZELLENPRÄPARAT UND KREBSTHERAPIEMITTEL MIT DEM MIZELLENPRÄPARAT ALS WIRKSTOFF
COPOLYMÈRE EN BLOCS POUR ADMINISTRATION INTRAPÉRITONÉALE CONTENANT UN AGENT ANTICANCÉREUX, PRÉPARATION DE MICELLE, ET AGENT DE TRAITEMENT DU CANCER COMPRENANT LA PRÉPARATION DE MICELLE EN TANT QU'INGRÉDIENT ACTIF

(30) Priority: 21.10.2009 JP 2009242238
(43) Date of publication of application: 29.08.2012
(73) Proprietor: Nippon Kayaku Kabushiki Kaisha, Tokyo 100-0005 (JP)
(72) Inventor: AKATSU Yuichi, Tokyo 115-8588 (JP); MASHIBA Hiroko, Tokyo 115-8588 (JP)
(74) Representative: Gille Hrabal
(86) International application number: PCT/JP2010/068268
(87) International publication number: WO 2011/049042

(56) References cited:
- EP-A1- 1 508 331
- EP-A1- 1 580 216
- WO-A1-2004/039869
- WO-A1-2006/033296
- WO-A2-2009/009067
- JP-A- 2001 072 606
- YANG, XIAO-QIANG ET AL.: 'FG020326-loaded nanoparticle with PEG and PDLLA improved pharmacodynamics of reversing multidrug resistance in vitro and in vivo.' ACTA PHARMACOLOGICA SINICA vol. 28, no. 6, 2007, pages 913 - 920, XP008155036
- DING, XUE-QIANG ET AL.: 'Antitumor effects of hydroxycamptothecin-loaded poly[ethylene glycol]poly[gamma-benzyl-L-glutamate] micelles against oral squamous' ONCOLOGY RESEARCH vol. 16, no. 7, 2007, pages 313 - 323, XP008097429
- ZHANG, XICHEN ET AL.: 'An investigation of the antitumor activity and biodistribution of polymeric micellar paclitaxel' CANCER CHEMOTHERAPY AND PHARMACOLOGY vol. 40, no. 1, 1997, pages 81 - 86, XP008155039
- ZHANG, X. ET AL.: 'Anti-tumor efficacy and biodistribution of intravenous polymeric micellar paclitaxel.' ANTI-CANCER DRUGS vol. 8, no. 7, 1997, pages 696 - 701, XP008155021
- YOKOYAMA, M. ET AL.: 'Polymer micelles as novel drug carrier: Adriamycin-conjugated poly (ethylene glycol)-poly(aspartic acid) block copolymer.' JOURNAL OF CONTROLLED RELEASE vol. 11, no. 1-3, 1990, pages 269 - 278, XP008155030
- HIROYUKI TSUJIMOTO ET AL.: 'DDS for intraperitoneal chemotherapy' DRUG DELIVERY SYSTEM vol. 22, no. 5, 2007, pages 522 - 529
- DEBORAH K. ARMSTRONG ET AL.: THE NEW ENGLAND JOURNAL OF MEDICINE, vol. 354, 2006, pages 34-43,

## Description

### Technical Field

The present invention relates to a therapeutic agent for treating cancer whose retention time is appropriate for intraperitoneal administration, comprising a micelle preparation using a block copolymer containing an anti-cancer agent.

### Background

Death rates of cancer have been increasing over the years in Japan. Among cancers, most of the progressed or recurrent cancers that are believed to have no complete cure available therefor, for example, stomach cancer, colorectal cancer, and ovarian cancer, are accompanied with peritoneal disseminated metastasis, which causes ileus or hydronephrosis. Peritoneal disseminated metastasis has thus damaged quality of life (QOL) .

From the viewpoint of a strategy for cancer treatment, it is extremely important to establish a therapeutic method for treating peritoneal disseminated metastasis, but peritoneal disseminated metastasis is difficult to treat. A chemotherapy, an intraperitoneal hyperthermic chemotherapy, and an intraperitoneal immunotherapy have been developed, but none of them is certainly established as a therapeutic method for peritoneal disseminated metastasis at present. With respect to the chemotherapy used for treating peritoneal disseminated metastasis caused by a cancer, an intravenously-administered anti-cancer agent is not well delivered to the abdominal cavity due to the blood peritoneum barrier, and the drug efficacy may not reach a sufficient level. A clinical trial based on an intraperitoneal chemotherapy has also been developed to promote drug efficacy of paclitaxel (cf. Non-Patent Literature 1). The intraperitoneal retention time however depends on each anti-cancer agent, and such effect cannot be fully obtained with an anti-cancer agent which is lost within a short period of time (cf. Non-Patent Literatures 2 and 3). Further, a hydrophobic anti-cancer agent requires a solubilizing agent which may cause a considerable side effect. To solve the issues above, it is essential to have a method of controlling intraperitoneal drug concentration for an extended period of time without using a solubilizing agent.

It has been known that a block copolymer of polyethylene glycols and polycarboxylic acid derivatives may combine with camptothecin via an ester bond to produce a polymer derivative (Patent Literature 1), and a micelle preparation may include encapsulated paclitaxel (Patent Literature 2). They showed a water solubility and a sustained release property for a long period of time in PBS and may maintain high blood drug concentration.

### Citation List

### Patent Literature

Patent Literature 1: International Publication No. WO/2004/039869 (corresponding to EP 1 580 216 A1)
Patent Literature 2: International Publication No. WO/2006/033296
Patent Literature 3: EP 1 508 331 A1

### Non Patent Literature

Non-Patent Literature 1: Deborah K. Armstrong et al. , The New England Journal of Medicine, 2006, Vol. 354, pp. 34-43.
Non-Patent Literature 2: Jones AI et al., Annals of Oncology, 1994, Vol. 5, pp. 123-126.
Non-Patent Literature 3: Atiq OT et al., Journal of Clinical Oncology, 1993, Vol. 11, pp. 425-433.

### Summary of Invention

### Technical Problem

The present invention provides a therapeutic agent for treating cancer which is appropriate for intraperitoneal administration of an anti-cancer agent. The agent enables a long term retention of the anti-cancer agent in the abdominal cavity.

It is preferred to control the intraperitoneal drug concentration for an extended period of time without using a solubilizing agent to avoid an adverse side effect of a solubilizing agent used for dissolving a hydrophobic anti-cancer agent.

### Solution to Problem

The present inventors have made extensive study to solve the problems above, and found that a micelle preparation comprising a block copolymer bonding to or encapsulating 7-ethyl-10-hydroxycamptothecin (SN-38) or paclitaxel as an anti-cancer agent may be intraperitoneally administered such that the anti-cancer agent may be retained in the abdominal cavity for an extended time period. A superior life-prolonging effect has been obtained in a mouse model for peritoneal disseminated metastasis compared to a case in which the anti-cancer agent was intravenously administered. The present invention has been completed accordingly.

Specifically, the present invention relates to the following items (1) and (2).
(1) A micelle preparation containing an anti-cancer agent,
   the preparation exhibiting sustained drug release capability, and enabling an extension of a retention time period of the anti-cancer agent in the abdominal cavity, comprising:
   a copolymer having a hydrophilic polymeric moiety and a polycarboxylic acid derivative moiety represented by the formula (1): 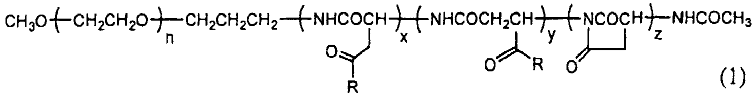 where
      n is an integer of from 100 to 300,
      x or y is an integer of 1 or more,
      z is an integer of 1 or more,
      (x + y + z) is an integer of from 10 to 80,
      the ratio of x to (x + y + z) is 0 to 90%,
      the ratio of y to (x + y + z) is 0 to 90%,
      the ratio of z to (x + y + z) is 1 to 80%, and
      R is a combination of one or more selected from hydroxy, 4-phenyl-l-butoxy, and isopropylaminocarbonyl-isopropylamino groups, with the proviso that the ratio of the hydroxy group to (x + y + z) is 0 to 10%, the ratio of the 4-phenyl-1-butoxy group to (x + y + z) is 10 to 90%, and the ratio of the isopropylaminocarbonyl-isopropylamino group to (x + y + z) is 5 to 30%, and
      an anti-cancer agent bonding to or encapsulated in the copolymer; or
   a polymeric derivative drug derived from camptothecins, represented by formula (2): 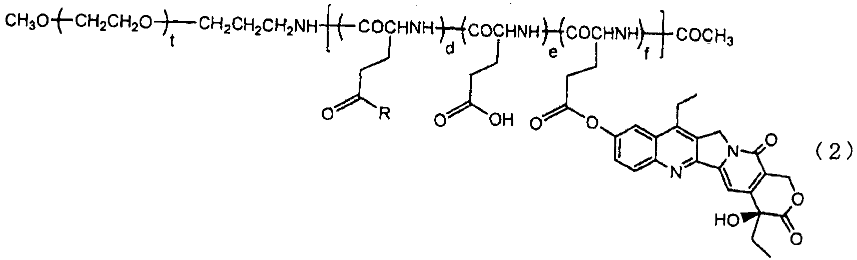 where
      t is an integer of from 100 to 300,
      (d + e + f) is an integer of from 6 to 60,
      the ratio of d to (d + e + f) is 0 to 60%,
      the ratio of e to (d + e + f) is 0 to 60%,
      the ratio of f to (d + e + f) is 1 to 100%, and
      R is an isopropylaminocarbonyl-isopropylamino group;
      for use in the prevention and treatment of peritoneal disseminated metastasis, or the treatment of cancers occurring in an organ present in the abdominal cavity, stomach cancer, colorectal cancer, pancreatic cancer, liver cancer, gall bladder cancer, ovarian cancer, uterine cancer, kidney cancer, ureteral cancer, or peritoneal cancer,
      by intraperitoneal administration.
(2) The micelle preparation according to item (1), wherein the anti-cancer agent which is bonded to or encapsulated in copolymer of the formula (1) is paclitaxel.

### Advantageous Effects of Invention

The present micelle preparation for intraperitoneal administration, which includes a block copolymer bonding to or encapsulating an anti-cancer agent, has an advantageous effect of extending the abdominal retention time of the anti-cancer agent, and therefore may be effectively used for treating cancers occurring in an organ present in the abdominal cavity or accompanying peritoneal disseminated metastasis which is difficult to treat. In addition, the present micelle preparation may exert sustained drug release capability without a solubilizing agent, and therefore it is expected to be used as a therapeutic agent for intraperitoneal administration with less adverse side-effects.

### Brief Description of Drawings

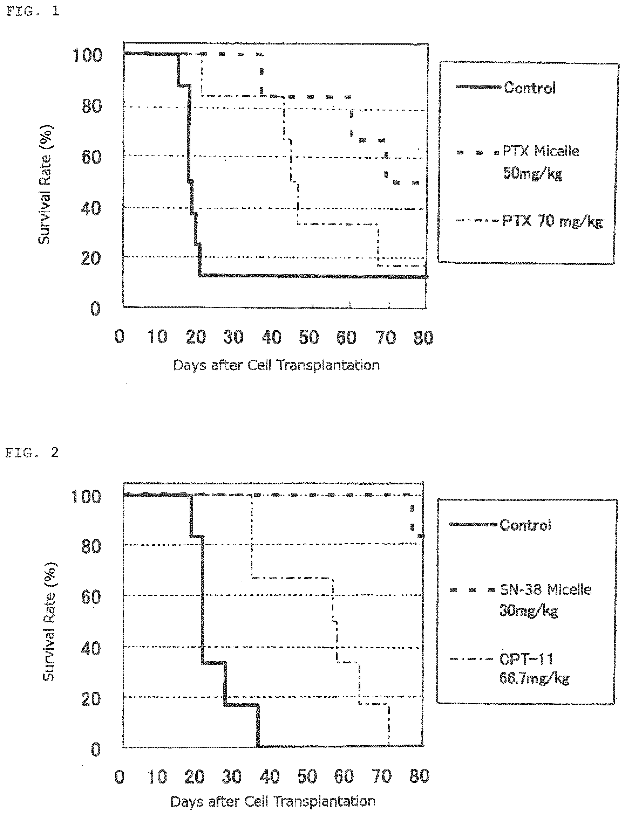

FIG. 1 illustrates an effect of paclitaxel (PTX) and a micelle preparation encapsulating PTX on a nude mouse model for peritoneal disseminated metastasis with human ovarian cancer cell line ES-2. The vertical axis represents the survival rate and the horizontal axis represents the days after transplantation.
FIG. 2 illustrates an effect of irinotecan hydrochloride (CPT-11) and a micelle preparation bonding to SN-38 which is an active metabolite of CPT-11 on a nude mouse model for peritoneal disseminated metastasis with human ovarian cancer cell line ES-2. The vertical axis represents the survival rate and the horizontal axis represents the days after transplantation.

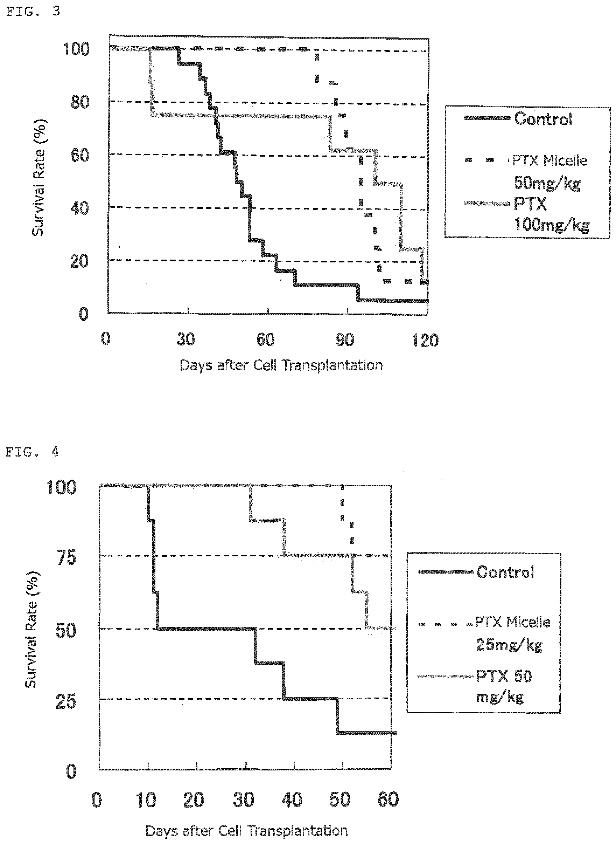

FIG. 3 illustrates an effect of paclitaxel (PTX) and a micelle preparation encapsulating PTX on a nude mouse model for peritoneal disseminated metastasis with human ovarian cancer cell line SHIN-3. The vertical axis represents the survival rate and the horizontal axis represents the days after transplantation.
FIG. 4 illustrates an effect of paclitaxel (PTX) and a micelle preparation encapsulating PTX on a nude mouse model for peritoneal disseminated metastasis with human stomach cancer cell line MKN45-P. The vertical axis represents the survival rate and the horizontal axis represents the days after transplantation.

### Description of Embodiments

According to the present invention, the copolymers of a hydrophilic polymeric moiety and a polycarboxylic acid derivative moiety as defined in claim 1 and claim 3 exhibit the required properties. The copolymer includes a copolymer having polyethylene glycols as a hydrophilic copolymer and polycarboxylic acid derivative.
The copolymer may be prepared by a process described in a conventional article, e.g., Patent Literature 1, 2 or 3, Japanese Patent Application Laid-Open No. 06-206815, Japanese Patent Application Laid-Open No. 2003-504393.

The anti-cancer agent, which is bound to or encapsulated in the block copolymer, may be any anti-cancer agents generally used for cancer treatment. The anti-cancer agent may include, but not limited to, taxoid-based drug, platinum drug, nitrosourea-based drug, nitrogen mustard-based drug, triazine-based drug, anthracycline-based drug, vinca alkaloid-based drug, epipodophyllotoxin-based drug, camptothecin-based drug, and fluoropyrimidine-based drug. The taxoid-based drug may include taxol, taxotere, paclitaxel, and docetaxel. The platinum-based drug may include cisplatin and carboplatin. The nitrosourea-based drug may include carmustin and lomustin. The nitrogen mustard-based drug may include cyclophosphamide. The triazine-based drug may include dacarbazine. The anthracycline-based drug may include doxorubicin. The vinca alkaloid-based drug may include vincristine and vinblastine. The epipodophyllotoxin-based drug may include etoposide. The camptothecin-based drug may include irinotecan. The fluoropyridine-based drug may include tegafur. A preferred anti-cancer agent may include paclitaxel and 7-ethyl-10-hydroxycamptothecin (SN-38) which is an active metabolite of irinotecan. Irinotecan is a water soluble prodrug, and converts into active metabolite SN-38 to exhibit an anti-tumor activity by a hydrolysis mainly with liver carboxyesterase after administration.

The method for binding or encapsulating an anti-cancer agent to/in the present block copolymer may be carried out in view of the Patent Literatures above.

With regard to a micelle preparation including a block copolymer to which an anti-cancer agent is bound, the amount of the agent may appropriately be varied from low concentration to high concentration.

In the case that a micelle preparation comprises a block copolymer in which an anti-cancer agent is encapsulated, the ratio of the amount of the anti-cancer agent to the total weight of the anti-cancer agent and the block copolymer is 0.1 % to 50 % by weight. Further, the drug content may be about 0.01 mg or more, and preferably about 0.1 mg or more by 1 ml of an aqueous solution of the micelle preparation.

A cancer which is an object of the treatment in the present therapeutic agent is selected from a cancer occurring in an organ present in an abdominal cavity such as stomach cancer, colorectal cancer, pancreatic cancer, liver cancer, gall bladder cancer, ovarian cancer, uterine cancer, kidney cancer, ureteral cancer, and peritoneal cancer. The present therapeutic agent may be intraperitoneally administered to effectively treat or prevent a recurrence of those cancers and peritoneal disseminated metastasis complicated with the cancers.

The term "peritoneal disseminated metastasis" indicates that visible particulate nodes or lumps are formed on the peritoneum. The nodes or lumps may occur by repeated divisions and proliferations of cancer cells distributed on the peritoneum, that are released from a surface of a cancer in an abdominal organ.

It is considered that progressed or recurred stomach cancer is often accompanied with peritoneal disseminated metastasis, and it would make the cancer treatment difficult. Further, it is known that peritoneal disseminated metastasis may be also accompanied with colorectal cancer, ovarian cancer, uterine cancer, gall bladder cancer, and ureteral cancer, as well as stomach cancer.

The present therapeutic agent is administered intraperitoneally. The term "intraperitoneal administration" means that a drug is administered directly into the abdominal cavity. The intraperitoneal administration of a drug may be carried out by injection or by using a catheter, for example.

Dosage of an anti-cancer agent may vary depending on the type of an anti-cancer agent used. The dosage may preferably be appropriately chosen and determined in view of the dosage that is used for general treatment. In the case that a micelle preparation containing paclitaxel or SN-38 (an active metabolite of irinotecan) is applied via intraperitoneal administration, its dosage may generally be about 0.1 to 500 mg/m² (body surface area) as an active component per day for an adult patient. Dosage may vary depending on age, symptom, or the like of a patient.

The present therapeutic agent may be prepared with various common additive components. The additive may include, but not limited to, a stabilizing agent, a bactericidal agent, a buffer agent, an isotonizing agent, a chelating agent, a pH controlling agent, a surfactant, and a solubilizing agent.

The stabilizing agent may include, but not limited to, human serum albumin, common L-amino acids, sugars, and cellulose derivatives. The stabilizing agent may be used either singly or in combination with a surface active agent or the like. According to the use of the combination, the stability of the effective component may be further improved. The L-amino acids are not particularly limited, and examples thereof may include glycine, cysteine, and glutamic acid. The sugars may include, but not limited to, monosaccharides such as glucose, mannose, galactose, and fructose; sugar alcohols such as mannitol, inositol, and xylitol; disaccharides such as sucrose, maltose, and lactose; and polysaccharides such as dextran, hydroxypropyl starch, chondroitin sulfate, and hyaluronic acid; and derivatives thereof. The cellulose derivatives may include, but not limited to, methyl cellulose, ethyl cellulose, hydroxyethyl cellulose, hydroxypropyl cellulose, hydroxylpropylmethyl cellulose, and sodium carboxymethyl cellulose. The surfactant may include, but not limited to, an ionic surface active agent and a non-ionic surface active agent, and may preferably include polyoxyethylene glycol sorbitan alkyl esters, polyoxyethylene alkyl ethers, sorbitan monoacyl esters, and fatty acid glycerides.

The buffer agent may include, but not limited to, boric acid, phosphoric acid, acetic acid, citric acid, ε-aminocaproic acid, glutamic acid, and/or salts thereof (e.g., an alkali metal salt such as sodium salt, potassium salt, calcium salt, and magnesium salt, and an alkali earth metal salt).

The isotonizing agent may include, but not limited to, sodium chloride, potassium chloride, sugars, and glycerin.

The chelating agent may include, but not limited to, edetate sodium and citric acid.

The solubilizing agent may include, but not limited to, alcohols such as ethanol.

### Examples

Hereinafter, the present invention is described in greater detail by reference to the Examples, but the present invention is not limited to the following examples.

### <Test Summary>

A micelle preparation, in which paclitaxel or SN-38 (an active metabolite of irinotecan hydrochloride) is encapsulated or bound, was used. An intraperitoneal administration of paclitaxel has been known as a significantly effective method for treating peritoneal disseminated metastasis caused by ovarian cancer compared to an intravenous administration (Non-Patent Literature 1). One of advantages of the intraperitoneal administration of paclitaxel relates to the fact that the intraperitoneal concentration may be higher than that of intravenous administration. However, although a direct effect of the intraperitoneal administration on peritoneal disseminated metastasis is expected, a drug is quickly lost from the abdominal cavity and therefore the concentration level will be reduced from the level required for exhibiting an anti-tumor effect after a short period of time. In this regard, a micelle preparation was prepared by binding a drug to a block copolymer or encapsulating it therein and administered to provide a sustained release capability. The consistency of the concentration required for exhibiting an anti-tumor effect and a superior life-prolonging effect obtained therefrom were estimated.

### <Test Protocol>

Therapeutic effects on peritoneal disseminated metastasis were tested by using a mouse model for peritoneal disseminated metastasis. Specifically, the mouse model for peritoneal disseminated metastasis was prepared by intraperitoneal injection of the human ovarian cancer cell line ES-2 (1.0 x 10⁶ cells) to an 8-week old BALB/c nude mouse. Note that the present therapeutic agent may apply to any other cancer cell lines other than ES-2.

A micelle preparation having sustained release capability in which paclitaxel is encapsulated was prepared according to the description of Examples 1 and 4 of Patent Literature 2 (International Publication No.2006/033296). The micelle preparation was used for present Test Example 1.

A micelle preparation having sustained release capability to which SN-38 is bound was prepared according to the description of the Example 1 of Patent Literature 1 (International Publication No. 2004/039869). The preparation was used for present Test Example 2.

### <Reference example 1: Production of micelle preparation having encapsulated paclitaxel>

A copolymer of formula (1) was synthesized according to the process disclosed in Example 1 of Patent Literature 2 (International Publication No. 2006/033296). 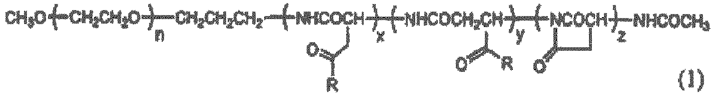 where n is about 272; (x + y + z) is 40; the ratio of (x + y) to (x + y + z) is 63 %; the ratio of z to (x + y + z) is 37 %; and for R, the ratio of the hydroxy group to (x + y + z) is 0 %, the ratio of 4-phenyl-1-butoxy group to (x + y + z) is 49 %, and the ratio of isopropyl aminocarbonyl-isopropylamino group to (x + y + z) is 14 %.

According to a process described in JP-A No. 06-206815, 42 g of PEG (average molecular weight 12000) -pAsp (polyaspartic acid; average polymerization number 40) -Ac (n is about 272, x is about 10, and y is about 30) was prepared, and DMF (630 ml), N,N-dimethyl aminopyridine (9.9 g), 4-phenyl-1-butanol (10.93 ml), and diisopropylcarbodiimide (15.86 ml) were added and maintained for 24 hours at 25°C. After purification of the reactants, about 48 g of block copolymer 1 was obtained. Block copolymer 1 (47.32 g) was dissolved in DMF (946 ml), added with N,N-dimethyl aminopyridine (7.23 g) and diisopropylcarbodiimide (14.37 ml), and maintained for 20 hours at 35°C. After purification of the reactants, about 44 g of target block copolymer 2 was obtained. The ratio of ester-bonded 4-phenyl-l-butanol was 49% to (x + y + z), and the ratio of hydroxy group was 0% to (x + y + z). The ratio of (x + y) to (x + y + z) was 63% and the ratio of z to (x + y + z) was 37 %.

Block copolymer 2 (300 mg) was added to 30 ml of an aqueous maltose solution (40 mg/ml) to make a dispersion, and then cooled to 4°C with stirring. 30 mg/ml paclitaxel of 3 ml dichloromethane solution was added, and stirred without sealing for 16 hours in a refrigerator. The mixture was subjected to an ultrasonication treatment at 130 W for 10 min to obtain a micelle preparation. The paclitaxel concentration was 2.2 mg/ml.

### <Reference example 2: Production of micelle preparation bound to SN-38>

A copolymer derivative of camptothecins of formula (2) was synthesized according to the process disclosed in Example 1 of Patent Literature 1 (International Publication No. 2004/039869). 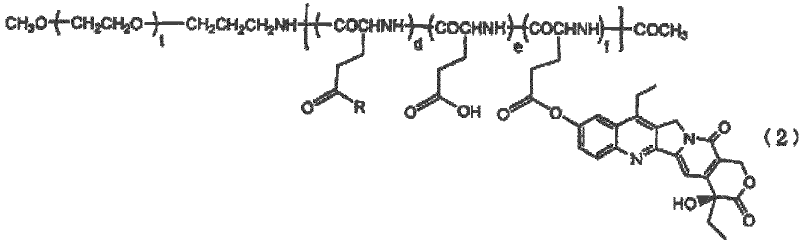 where t is about 273; (d + e + f) is about 28; the ratio of d to (d + e + f) is 15.5 %; the ratio of e to (d + e + f) is 36.1 %; the ratio of f d to (d + e + f) is 48.4 %; and R is an isopropylaminocarbonyl-isopropylamino group).

A block copolymer (210 mg) of methoxypolyethylene glycol (molecular weight: about 12,000) and polyglutamic acid (polymerization degree: about 28) and 7-ethyl-10-hydroxycamptothecin (80 mg) were dissolved in DMF (14 ml), added with N,N-dimethyl aminopyridine (13.5 mg) and diisopropylcarbodiimide (0.116 ml), and stirred for 20 hours at room temperature. After purification of the reactants, the target compound (270 mg) was obtained. The target compound had 7-ethyl-10-hydroxycamptothecin (25.4 w/w%) conjugate and an isopropylaminocarbonyl-isopropylamino group (3.0 w/w%) bound to the polyglutamic acid moiety. The ratio of d to (d + e + f) was 15.5 %, the ratio of e to (d + e + f) was 36.1 %, and the ratio of f to (d + e + f) was 48.4%.

### <Test example 1: Intraperitoneal administration of micelle preparation having encapsulated paclitaxel>

In order to estimate advantageous life-prolonging effects of the present micelle preparation having encapsulated paclitaxel, the model mice were divided into three groups, i.e., 8 mice per group. 1 ml of saline (Control group) ; 1 ml of saline containing 70 mg/kg of paclitaxel (70 mg/kg PTX administered group) ; and 1 ml of 5% glucose solution containing the micelle preparation containing 50 mg/kg of encapsulated paclitaxel (50 mg/kg PTX micelle administered group) were intraperitoneally administered to the mouse of each group, respectively. Intraperitoneal administrations were carried out once after three days of tumor transplantation. In addition, the drug dosage was determined by converting a clinical dosage for intravenous route (paclitaxel: 210 mg/m² and paclitaxel micelle preparation: 150 mg/m²) to those for a mouse using an AUC comparison. The mice were observed until 80 days after the cell transplantation. The survival rates were recorded and the survival curves were plotted in Fig. 1.

Superior life-prolonging effect of the present micelle preparation having encapsulated paclitaxel was estimated on the mouse model for peritoneal disseminated metastasis. The 50% survival rates of the Control group, the 50 mg/kg PTX micelle administered group, and the 70 mg/kg PTX administered group were on Day 17, Day 69, and Day 44 after the transplantations, respectively. The paclitaxel micelle preparation had a superior effect over paclitaxel.

### <Test example 2: Intraperitoneal administration of micelle preparation having bound SN-38>

In order to determine the superior life-prolonging effect of the micelle preparation having bound SN-38 (an active metabolite of irinotecan hydrochloride), the model mice were divided into three groups, i.e., 6 mice per group. 1 ml of saline (Control group); 1 ml of saline containing 66.7 mg/kg of irinotecan hydrochloride (66.7 mg/kg CPT-11 administered group) ; and 1 ml of 5% glucose solution containing the micelle preparation containing 30 mg/kg of SN-38 (30 mg/kg SN-38 micelle administered group) were intraperitoneally administered to the mouse of each group, respectively. The intraperitoneal administrations were carried out once after three days of tumor transplantations. In addition, the drug dosage was determined as the one-third of the maximum amount allowed for intravenous route administration for a mouse. The mice were observed until 80 days after the cell transplantation. The survival rates were recorded and the survival curves were plotted in Fig. 2.

Superior life-prolonging effect of the micelle preparation having bound SN-38 was estimated on a model for peritoneal disseminated metastasis. The 50% survival rate of the Control group was on Day 21 after the transplantation. The five-sixth of the mice in the 30 mg/kg SN-38 micelle administered group were survived for 80 days after the transplantation. The 50% survival rate of the 66.7 mg/kg CPT-11 administered group was on Day 56 after the transplantation. The SN-38 micelle preparation had a superior effect over irinotecan hydrochloride.

### <Test example 3: Intraperitoneal administration of micelle preparation having encapsulated paclitaxel>

In order to estimate an effect of intraperitoneal administration of the micelle preparation having encapsulated paclitaxel, a mouse model for peritoneal disseminated metastasis was prepared by intraperitoneal injection of human ovarian cancer cell line SHIN-3 (1.0 × 10⁷ cells) to an 8-week old BALB/c nude mouse. Three groups were created as following: 1 ml of saline (Control group, 18 mice) ; 1 ml of saline containing 100 mg/ml of paclitaxel (100 mg/kg PTX administered group, 8 mice); and 1 ml of 5% glucose solution containing the micelle preparation containing 50 mg/ml of encapsulated paclitaxel (50 mg/kg PTX micelle administered group, 8 mice) were intraperitoneally administered to the mouse of each group, respectively. The amounts of the ingredients were of by 20 g mouse body weight. The administrations were carried out once after one week of the tumor transplantations. The mice were observed until 120 days after the cell transplantation. The survival rates were recorded and the survival curves were plotted in Fig. 3.

The effect of the intraperitoneal administration of a micelle preparation having encapsulated paclitaxel was estimated. According to the intraperitoneal administration, the 50% survival rate of the 50 mg/kg PTX micelle administered group was on Day 95 after transplantation, and those of the 100 mg/kg PTX administered group was on Day 100 after transplantation. Their effects were similar in each group. There were two cases of toxic death recognized in the PTX administered group, while no toxic death was observed in the PTX micelle administered group at the dosage of 100 mg/kg as paclitaxel. The micelle preparation had no adverse effect and the same efficacy as paclitaxel at dosage which was half of those of paclitaxel showing toxicity. The intraperitoneal administration of a micelle preparation was certainly useful for treating peritoneal disseminated metastasis.

### <Test example 4: Intraperitoneal administration of micelle preparation having encapsulated paclitaxel>

In order to estimate an effect of intraperitoneal administration of a micelle preparation having encapsulated paclitaxel, a mouse model for peritoneal disseminated metastasis was prepared by intraperitoneal injection of human stomach cancer cell line MKN45-P (1.0 x 10⁶ cells) to an 8-week old BALB/c nude mouse. Three groups were created as following: 1 ml of saline (Control group, 8 mice) ; 1 ml of saline containing 50 mg/ml of paclitaxel (50 mg/kg PTX administered group, 8 mice); and 1 ml of 5% glucose solution containing the micelle preparation containing 25 mg/ml of encapsulated paclitaxel (25 mg/kg PTX micelle administered group, 8 mice) were intraperitoneally administered to the mouse of each group, respectively. The amounts of the ingredients were of by 25 g mouse body weight. The administrations were carried out once after three days of the tumor transplantation. The mice were observed until 60 days after the cell transplantation. The survival rate was recorded and the survival curves were plotted in Fig. 4.

An effect of the intraperitoneal administration of a micelle preparation having encapsulated paclitaxel was estimated. According to the intraperitoneal administration, the 50% survival rates of the 50 mg/kg PTX administered group and the 25 mg/kg PTX micelle administered group were on Day 55 and Day 60 or later after transplantation, respectively. Efficacy shown in the 25 mg/kg PTX micelle administered group was the same or higher than the paclitaxel group, even with half dosage. The intraperitoneal administration of the present micelle preparation having encapsulated paclitaxel was useful not only for human ovarian cancer but also for peritoneal disseminated metastasis associated with human stomach cancer.

### Industrial Applicability

The drug according to the present invention has an effect of extending retention time of an intraperitoneally administered anti-cancer agent in the abdominal cavity, and may provide an advantageous effect of the anti-cancer agent. The drug of the present invention can effectively treat a cancer which occurs in an abdominal organ and accompanying peritoneal disseminated metastasis which was difficult to treat. Thus, the applicability of the present invention is extremely high and useful in the field of medicine.

## Claims

1. A micelle preparation containing an anti-cancer agent,
the preparation exhibiting sustained drug release capability, and enabling an extension of a retention time period of the anti-cancer agent in the abdominal cavity, comprising:
a copolymer having a hydrophilic polymeric moiety and a polycarboxylic acid derivative moiety represented by the formula (1): 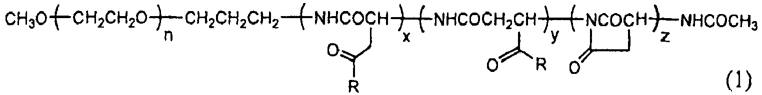 where
n is an integer of from 100 to 300,
x or y is an integer of 1 or more,
z is an integer of 1 or more,
(x + y + z) is an integer of from 10 to 80,
the ratio of x to (x + y + z) is 0 to 90%,
the ratio of y to (x + y + z) is 0 to 90%,
the ratio of z to (x + y + z) is 1 to 80%, and
R is a combination of one or more selected from hydroxy, 4-phenyl-1-butoxy, and isopropylaminocarbonyl-isopropylamino groups, with the proviso that the ratio of the hydroxy group to (x + y + z) is 0 to 10%, the ratio of the 4-phenyl-1-butoxy group to (x + y + z) is 10 to 90%, and the ratio of the isopropylaminocarbonyl-isopropylamino group to (x + y + z) is 5 to 30%, and
an anti-cancer agent bonding to or encapsulated in the copolymer; or
a polymeric derivative drug derived from camptothecins, represented by formula (2): 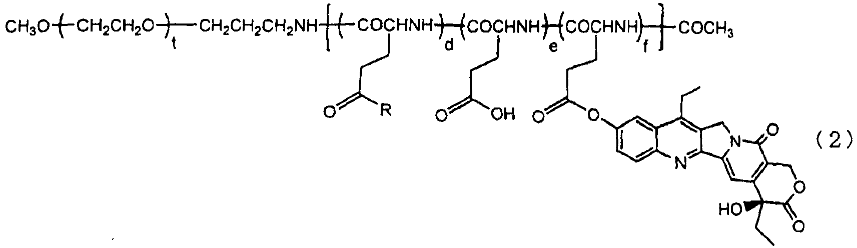 where
t is an integer of from 100 to 300,
(d + e + f) is an integer of from 6 to 60,
the ratio of d to (d + e + f) is 0 to 60%,
the ratio of e to (d + e + f) is 0 to 60%,
the ratio of f to (d + e + f) is 1 to 100%, and
R is an isopropylaminocarbonyl-isopropylamino group;
for use in the prevention and treatment of peritoneal disseminated metastasis, or the treatment of cancers occurring in an organ present in the abdominal cavity, stomach cancer, colorectal cancer, pancreatic cancer, liver cancer, gall bladder cancer, ovarian cancer, uterine cancer, kidney cancer, ureteral cancer, or peritoneal cancer,
by intraperitoneal administration.

2. The micelle preparation for use according to claim 1, wherein the anti-cancer agent which is bonded to or encapsulated in copolymer of the formula (1) is paclitaxel.

## Patentansprüche

1. Ein ein Antikrebsmittel enthaltendes Mizellenpräparat,
wobei das Präparat eine anhaltende Wirkstoff-Freisetzungsfähigkeit aufweist und eine Verlängerung einer Verweilzeit des Antikrebsmittels in der Bauchhöhle ermöglicht, umfassend:
ein Copolymer mit einem hydrophilen polymeren Anteil und einem Polycarbonsäure-Derivatanteil, dargestellt durch die Formel (1): 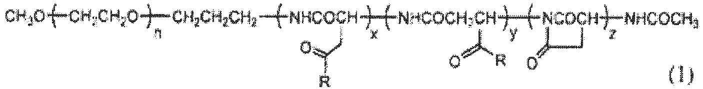 wobei
n eine ganze Zahl von 100 bis 300 ist,
x oder y eine ganze Zahl von 1 oder mehr ist,
z eine ganze Zahl von 1 oder mehr ist,
(x + y + z) eine ganze Zahl von 10 bis 80 ist,
das Verhältnis von x zu (x + y + z) 0 bis 90 % ist,
das Verhältnis von y zu (x + y + z) 0 bis 90 % ist,
das Verhältnis von z zu (x + y + z) 1 bis 80 % ist und
R eine Kombination aus einer oder mehreren ist, ausgewählt aus den Hydroxy-, 4-Phenyl-1-butoxy- und Isopropylaminocarbonyl-isopropylaminogruppen, mit der Maßgabe, dass das Verhältnis der Hydroxygruppe zu (x + y + z) 0 bis 10 % beträgt, das Verhältnis der 4-Phenyl-1-butoxygruppe zu (x + y + z) 10 bis 90 % beträgt und das Verhältnis der Isopropylaminocarbonyl-isopropylaminogruppe zu (x + y + z) 5 bis 30 % beträgt, und
ein Antikrebsmittel, das an das Copolymer gebunden oder in dieses eingekapselt ist; oder einen polymeren Derivat-Wirkstoff, der von Camptothecinen abgeleitet ist, dargestellt durch die Formel (2): 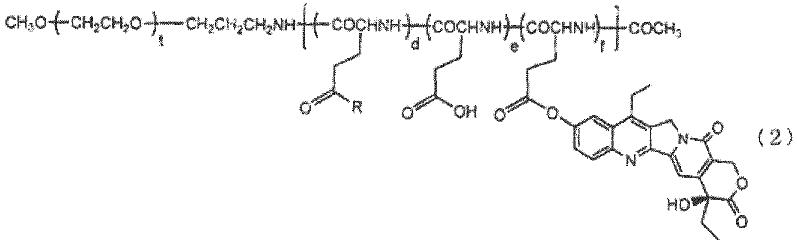 wobei
t eine ganze Zahl von 100 bis 300 ist,
(d + e + f) eine ganze Zahl von 6 bis 60 ist,
das Verhältnis von d zu (d + e + f) 0 bis 60 % beträgt,
das Verhältnis von e zu (d + e + f) 0 bis 60 % beträgt,
das Verhältnis von f zu (d + e + f) 1 bis 100 % beträgt und
R eine Isopropylaminocarbonyl-isopropylaminogruppe ist;
zur Verwendung bei der Prävention und Behandlung von peritonealen disseminierten Metastasen oder zur Behandlung von Krebserkrankungen eines in der Bauchhöhle vorhandenen Organs, Magenkrebs, Darmkrebs, Bauchspeicheldrüsenkrebs, Leberkrebs, Gallenblasenkrebs, Eierstockkrebs, Gebärmutterkrebs, Nierenkrebs, Harnleiterkrebs oder Peritonealkrebs,
durch intraperitoneale Verabreichung.

2. Das Mizellenpräparat zur Verwendung nach Anspruch 1, wobei das Antikrebsmittel, das an ein Copolymer der Formel (1) gebunden oder eingekapselt ist, Paclitaxel ist.

## Revendications

1. Préparation de micelle contenant un agent anticancéreux,
la préparation présentant une capacité de libération prolongée de médicament, et permettant de prolonger une durée de rétention de l'agent anticancéreux dans la cavité abdominale, comprenant :
un copolymère ayant une partie polymère hydrophile et une partie de dérivé d'acide polycarboxylique représenté par la formule (1) : 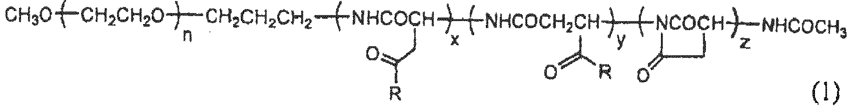 où
n représente un nombre entier allant de 100 à 300,
x ou y représente un nombre entier supérieur ou égal à 1,
z représente un nombre entier supérieur ou égal à 1,
(x + y + z) représente un nombre entier allant de 10 à 80,
le rapport de x sur (x + y + z) va de 0 à 90%,
le rapport de y sur (x + y + z) va de 0 à 90%,
le rapport de z sur (x + y + z) va de 1 à 80%, et
R représente une combinaison d'un ou de plusieurs groupe(s) choisi(s) parmi des groupes hydroxy, 4-phényl-1-butoxy, et isopropylaminocarbonyl-isopropylamino, à condition que le rapport du groupe hydroxy sur (x + y + z) soit allant de 0 à 10%, le rapport du groupe 4-phényl-1-butoxy sur (x + y + z) soit allant de 10 à 90%, et le rapport du groupe isopropylaminocarbonyl-isopropylamino sur (x + y + z) soit allant de 5 à 30%, et
un agent anticancéreux se liant au copolymère ou encapsulé dans celui-ci ; ou
un médicament à base de dérivé polymère, dérivé de camptothécines, représenté par la formule (2) : 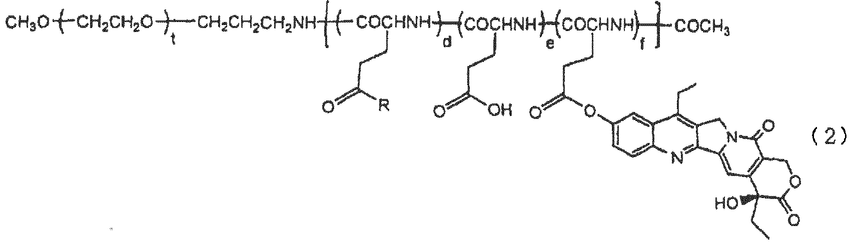 où
t représente un nombre entier allant de 100 à 300,
(d + e + f) représente un nombre entier allant de 6 à 60,
le rapport de d sur (d + e + f) va de 0 à 60%,
le rapport de e sur (d + e + f) va de 0 à 60%,
le rapport de f sur (d + e + f) va de 1 à 100%, et
R représente un groupe isopropylaminocarbonyl-isopropylamino ;
pour une utilisation dans la prévention et le traitement de métastase péritonéale disséminée, ou le traitement de cancers survenant dans un organe présent dans la cavité abdominale, le cancer de l'estomac, le cancer colorectal, le cancer du pancréas, le cancer du foie, le cancer de la vésicule biliaire, le cancer de l'ovaire, le cancer de l'utérus, le cancer du rein, le cancer de l'urètre ou le cancer péritonéal,
par administration intrapéritonéale.

2. Préparation de micelle pour une utilisation selon la revendication 1, dans laquelle l'agent anticancéreux qui est lié à un copolymère de la formule (1) ou encapsulé dans celui-ci est le paclitaxel.
